# EUROPEAN PATENT SPECIFICATION

(11) **EP 1 765 203 B1**
(45) Date of publication and mention of the grant of the patent: **12.12.2007**
(21) Application number: 05750333.6
(22) Date of filing: 29.06.2005
(51) Int. Cl.: A61B 17/58, A61B 17/68

(54) **DEVICE FOR FASTENING POST-CRANIOTOMY BONE FLAPS**
VORRICHTUNG ZUR BEFESTIGUNG VON KNOCHENLAPPEN NACH EINER KRANIOTOMIE
DISPOSITIF DESTINE A FIXER DES VOLETS OSSEUX APRES UNE CRANIOTOMIE

(30) Priority: 05.07.2004 ES 200401631
(43) Date of publication of application: 28.03.2007
(73) Proprietor: Neos Surgery, S.L., 08290 Cerdanyola del Valles (ES)
(72) Inventor: GILETE GARCIA, Vicente, E-08025 Barcelona (ES)
(74) Representative: SUGRANES - VERDONCES - FERREGÜELA
(86) International application number: PCT/EP2005/006267
(87) International publication number: WO 2006/002744

(56) References cited:
- WO-A-02/09602
- FR-A- 2 794 019
- US-A1- 2001 011 173
- DATABASE WPI Section PQ, Week 200356 Derwent Publications Ltd., London, GB; Class P31, AN 2003-594336 XP002342798 & JP 2003 220070 A (KANAI H) 5 August 2003 (2003-08-05)

## Description

The present invention relates to a device for fastening bone flaps, which have been separated from a cranial bone mass during a surgical operation, and wherein at least one cranial orifice is defined, the device comprising external support means to the outer edges of the cranial bone mass and the bone flap; and internal support means for the inner edges of the cranial bone mass and the bone flap.

Many devices for fastening bone fragments or flaps are known in the field of Neurosurgery, which generally have means for fastening the flaps, and suitable for supporting or holding the cranial bone mass in a space set aside for such purpose, or by making an incision with a piercing element in the soft area of said cranial mass. These devices are positioned between the flap and the cranial bone and, except in cases where they are made of biodegradable material, remain permanently in the cranium.

Some examples of devices for fastening flaps for craniotomy operations are those described in Patents US 5,916,217; ES 2184639; and ES 2199085. All of these patents disclose metallic devices with a simple configuration, useful for their purpose, based on holding the flap between two elements that define a cavity, and being supported, via a plate or other item similar to the cranial bone mass, or penetrating the soft area of the bone using small tongues.

Another type of devices for fastening flaps generally consists in two essentially circular elements, facing one another and joined via a rod, whereby hemispherical elements can be moved with respect to each other in order to adjust the device to the thickness of the flap and cranium bone mass. Once the device has been adjusted, so that both the flap and the cranial bone mass are supported between the hemispherical elements, the neurosurgeon cuts off the rest of the remaining rod. This type of device has the drawback that it is difficult to use since, once adjusted, the remaining rod must be cut off.

Due to the progress of studies on new materials, and the existence of materials called shape-memory materials, this type of material is being applied in different fields of medicine. The shape-memory effect can be described as the capacity of a material to change its form due to the application of an external stimulus.

The known shape-memory materials could be: metal alloys; shape-memory polymers; shape-memory ceramics and shape-memory ferromagnetic alloys. These classes of material are differentiated by their nature, the material itself or the external stimulus to which they respond. In the case of metal alloys, the shape-memory effect is based on the transition that occurs between two solid phases, one low temperature or martensitic and the other high temperature or austenitic. The shape-memory properties of polymers are produced by their structure and programming of the desired shape. Generally, the shape-memory effect in polymers is produced by temperature, light or chemical reactions. In the case of ceramics that possess these qualities, their properties are usually based on Zr02 or magnesium niobate. Lastly, shape-memory ferromagnetic alloys behave in a similar way to the metal alloys described earlier, but respond to magnetic stimuli.

The first medical field wherein said materials were found to have a beneficial application, was the field of vascular medicine, but they are also now being applied in the field of bone prostheses, especially for repairing-fractures and correcting defects in the spine.

Examples wherein devices made of shape-memory materials are used in bone mass are those disclosed in Patents DE 4210801 and FR 2718634. In Patent DE 4210801, a procedure for manufacturing a nickel-titanium alloy is disclosed which is useful for bone repair or prostheses and which may remain in the body once used. In this patent, the use of said alloy in devices for joining bone fragments is described, but its application for fastening bone flaps is not specified. Patent FR 2718634 also discloses a shape-memory implant whose martensitic transformation takes place at a temperature below that of the human body, and which has a "w"-shaped configuration, for use, in particular, with femur, knee, foot and hand prostheses, for strengthening a bone. Lastly, Patent US 5,964,770 also claims a medical device comprised of a shape-memory material which is capable of adopting two functional positions at the basal temperature of the human body. Although this patent describes the use of devices for repairing bone defects or fractures, it neither shows nor implies an application for fastening craniotomy bone flaps.

The present invention proposes an original solution to the problems that arise, which also brings many benefits in the field of Neurosurgery, especially in operations wherein, once the operation is over, bone flaps must be returned to the structures from which they were removed.

The device for fastening postcranial bone flaps being the object of the invention is characterized in that the external support means and internal support means are joined via an intermediate element made of a shape-memory material, with an initial configuration obtained by subjecting said element to certain determined requirements, and that once deformed in order to apply the device between the flap and the cranial bone mass, and upon certain new requirements being applied to it, it adopts the initial configuration.

According to another characteristic of the invention, the external support means and internal support means consist of two platforms in the form of disks, which are positioned parallel to one another and joined by a strip of shape-memory material which passes through cuts made in the platforms.

The device for fastening bone flaps being the object of the invention, is also characterized in that it consists of two bands of shape-memory material, joined by a tangential line shared by both bands, outlining two opposite cavities for receiving the respective edges of the bone flap and cranial bone mass.

The device for fastening bone flaps is also characterized in that the external support means and internal support means consist of two platforms, which are positioned parallel to one another and joined by a spring made of a shape-memory material, whose ends are adapted to receive respective projections made in the inner surface of the platforms.

The attached drawings illustrate a non-restrictive example of a device for fastening flaps in accordance with the invention. In said drawings:
- Fig. 1: corresponds to a perspective view of the device according to the invention in a deformed position ready for insertion between the bone flap and the cranial bone mass.
- Fig. 2: is the same perspective view as in Fig. 1, but in the initial or final operational configuration;
- Fig. 3: corresponds to a longitudinal cross-section of the device in Fig. 2;
- Figs. 4, 5 and 6: correspond to other examples of devices for fastening bone fragments according to the invention;
- Fig. 7: corresponds to a perspective view of a device according to the invention which is very similar to that of Figs. 1 to 3, in a deformed position ready for insertion between the bone flap and the cranial bone mass;
- Fig. 8: is the same perspective view as in Fig. 7, but in the initial or final configuration;
- Fig. 9: corresponds to a longitudinal cross-section of the device in Fig. 8;
- Fig. 10: shows a longitudinal cross-section of another device according to the invention; and
- Fig. 11: corresponds to an elevation view of another device according to the invention.

In the attached drawings, a device for fastening bone flaps or fragments 1 can be seen, which comprises external support means 2 to the upper edges 3-3' of a cranial bone mass 4 and a bone flap 5; and internal support means 6 for the lower edges 7-7' of the cranial bone mass 4 and the bone flap 5. Said external 2 and internal support means 6 are joined via an intermediate element 8 which consists of a shape memory material. With a device 1 of this type, the user can deform it from an initial configuration obtained by subjecting said element to determined requirements, and can thus apply it between the cranial bone mass 4 and the bone flap 5, which has been separated in order to be able to perform a neurosurgical operation. Subsequently, due to the properties presented by shape-memory materials, the device 1, on being subjected to certain requirements, regains its initial configuration.

The user or neurosurgeon then, therefore, mechanically deforms the device 1 and, once inserted between the bone flap 5 and cranial bone mass 4, subjects it to the requirements which enable the external support means 2 and internal support means 6 to come into contact with the cranial bone mass 4 and the bone flap, pressing against their edges 3-3' and 7-7'.

Said conditions or requirements often refer to a determined range of temperatures which includes the human basal temperature. In this way, the initial configuration of the intermediate shape-memory element 8 is produced by subjecting the material to a determined range of temperatures of about 37°C and, once applied, the device 1 recovers its initial configuration upon coming in contact with the human body. The surgeon can also help to encourage its return to the initial configuration by applying small amounts of tepid saline solution.

Preferred materials for making up the intermediate element 8 are metal alloys; shape-memory polymers; shape-memory ceramics and shape-memory ferromagnetic alloys. A nickel-titanium alloy, known as "nitinol", is preferably used which, in addition to being a bio-compatible alloy, recovers the initial or final configuration via the external action of a temperature within a range that includes body temperature. All these materials known as shape-memory materials are also characterized in that they are so elastic that they can be very easily deformed by the mechanical action of a surgeon, and return to their initial condition once the force that deformed them is remove and/or if certain determined external requirements or conditions are applied under which they recover their initial or final operational configuration. These shape-memory materials are generally treated with low temperatures in order to provide them with a determined configuration. Subsequently, once deformed mechanically, for example, because of their own inherent properties and atomic distribution, they can return to their initial configuration when subjected to the same or other temperature and/or pressure conditions.

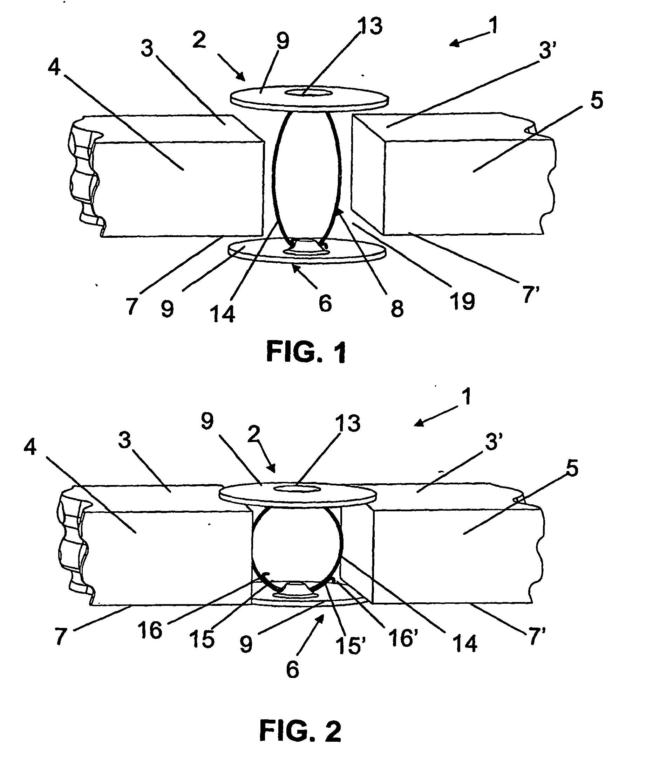

Figs. 1 and 2 show a device for fastening bone fragments 1 according to the invention, wherein two platforms 9 with a concave part 13 wherein cuts are made 10, are joined via an intermediate element 8 which consists of a strip 14 which passes through the cuts 10 in the platforms. Said strip 14 has on its opposite free ends 15-15' two hooks 16-16' which prevent the intermediate element 8 from escaping from the cuts 10. More specifically, in Fig. 1, the device 1 is shown in its position for use, once it has been deformed or stretched by the surgeon. Alternatively, in Fig. 2, the device 1 appears in its functional position or initial and/or final configuration, wherein the external support means 2 and internal support means 6 come into contact with the cranial bone mass 4 and the bone flap 5, pressing on their outer 3-3' and inner edges 7-7'.

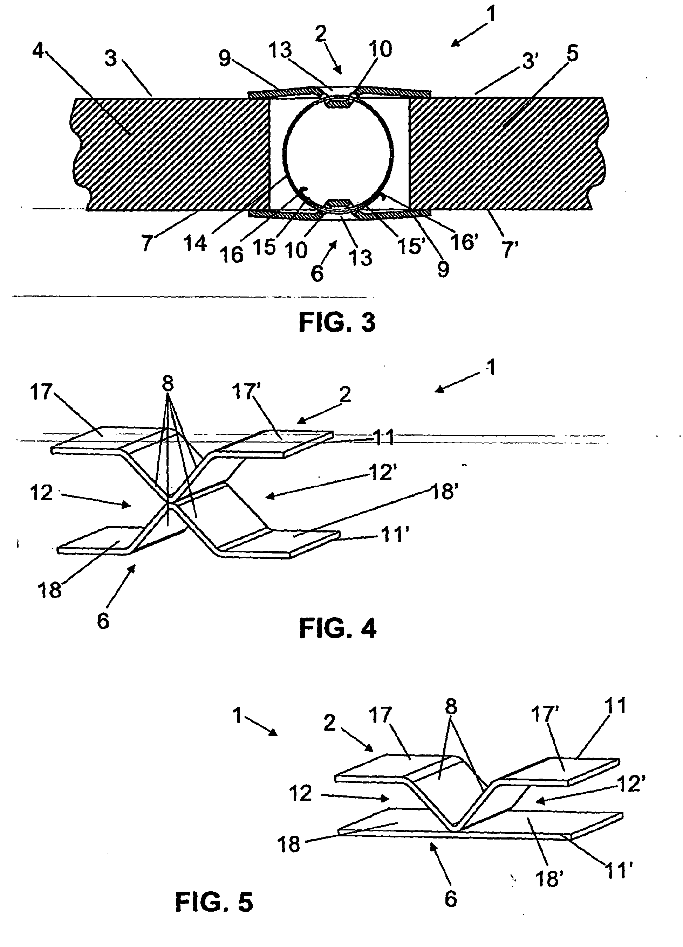

With the aim of providing a better example of the device 1 as shown in Figs. 1 and 2, in Fig. 3 shows a longitudinal cross-section of the device in Fig. 2, wherein it can clearly be seen that a strip 14 used as an intermediate shape-memory element 8 has been inserted through the cuts 10 of the platforms in the form of disks 9 which exert pressure against the outer 3-3' and inner edges 4-4', the strip regaining its initial or final operational configuration.

The platforms 9, which in this case are in the form of a disk, can be made of any bio-compatible material, and platforms using titanium or titanium alloys, bio-compatible polymers, such as polyetheretherketone (PEEK) or shape-memory materials themselves are therefore used.

Obviously, the device 1 may entirely consist of a shape-memory material. In this way, the entire device has the properties for being easily deformed by the mechanical action of a surgeon, and to return to its initial operational condition when determined external requirements are applied to it.

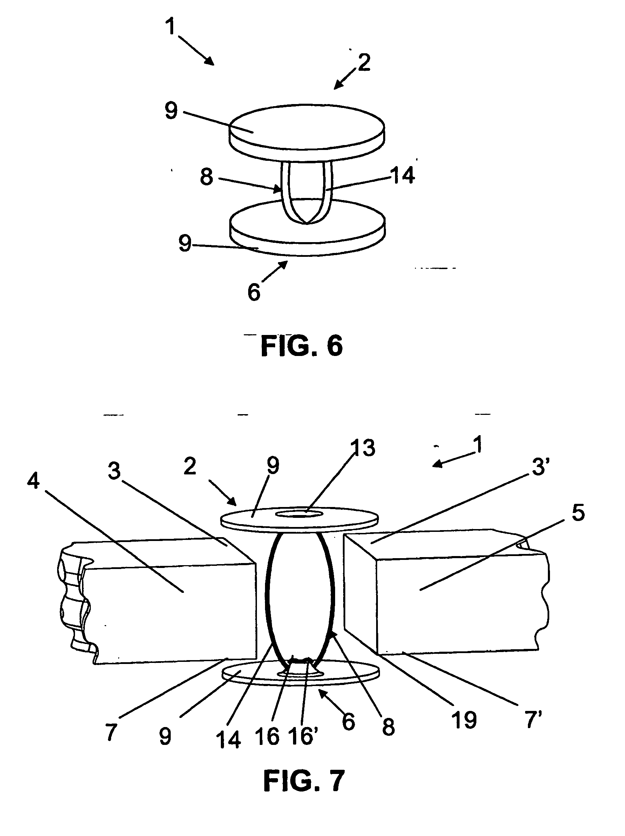

A very similar alternative to the one shown in Figs. 1 to 3, is that given in Fig. 6, wherein two platforms in the form of disks 8 are joined by a continuous strip 14 which forms part thereof.

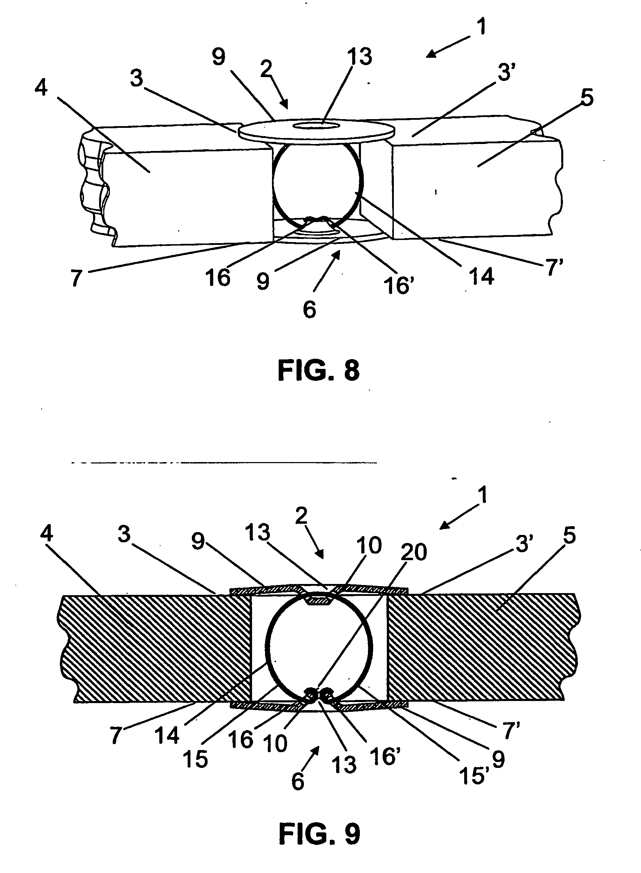

In the same way as in Figs. 1 to 3, Figs. 7 to 9 show a device for fastening bone flaps 1, wherein the hooks 16 and 16' on the ends 15 and 15' of the strip 14, are positioned through some cuts 10 made in the platforms 9, and are secured at the ends in a second hole 20, located in the base of one of the cavities 13 of the inner platform 9.

The devices for fastening flaps 1 as shown in Figs. 4 and 5 also form part of the object of the invention.

More specifically, in Fig. 4, the device 1 consists of two bands of shape-memory material 11-11', which have been given an "X" configuration and which determine two opposite cavities 12-12', which on one side house the respective edges of the bone flap 5 and, on the other, those of the cranial bone mass 4, or cranium. In this specific case, the upper support means 2 are defined by the two ends 17-17' of the outer band 11, while the support means 6 are defined by the two ends 18-18' of the inner band 11'.

In an alternative form, in the device for fastening flaps 1 shown in Fig. 5, the outer band 11 is the same as the one described for the representation in Fig. 4, but the inner band 11' consists of a flat band.

In both the device 1 of Fig. 4 and that of Fig. 5, the intermediate shape-memory element 8 that joins the external support means 2 and internal support means 6, are defined by a section of at least one of the bands of shape-memory material 11-11'. The particular form of the devices, essentially in the form of an "X", for fastening bone fragments 1 facilitates their mechanical deformation by the surgeon in order to insert them in the intraosseous orifice or space 19, i.e. between the flap 5 and the cranial bone mass 4.

Obviously, any of the devices for fastening bone flaps 1 represented in Figs. 1 to 6 may be used, not just in the groove 19 between the flap 5 and the cranial bone mass 4, but also, and especially, those described in Figs. 1, 2, 3 and 6 to 10, for sealing craniectomy and trepanation orifices.

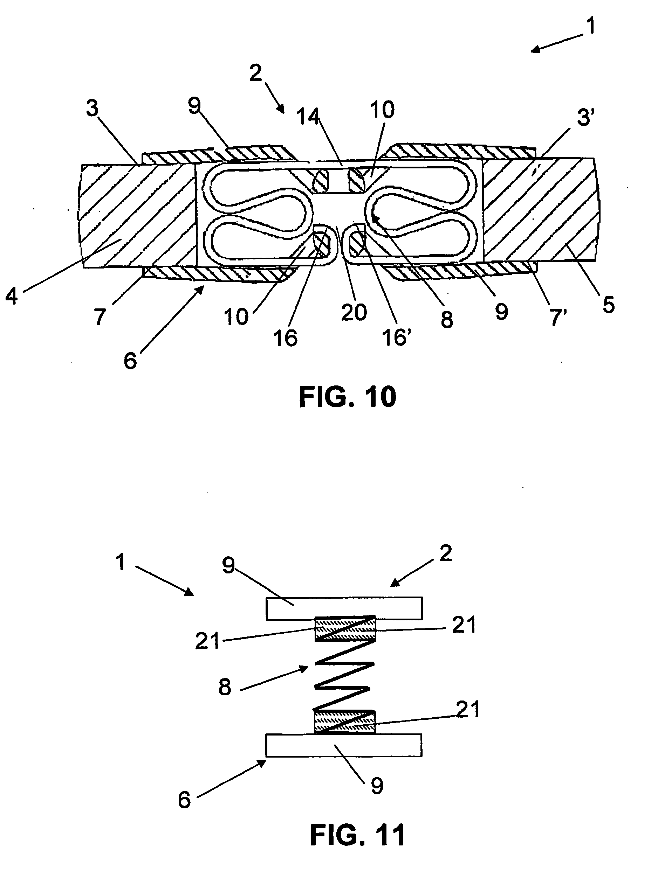

Said intermediate element 8 may obviously adopt any form and configuration, as can be seen in Fig. 10 or, for example, may have the form of a spring as shown in Fig. 11. The condition required to make it functional is that the elastic material it is made of is of the group of materials known as shape-memory materials.

## Claims

1. Device for fastening bone flaps (1), used to secure bone fragments or flaps (5) separated from a cranial bone mass (4) during a surgical operation wherein at least one cranial orifice (19) is defined, the device comprising some external support means (2) to the outer edges (3, 3') of the cranial bone mass and the bone flap; and some internal support means (6) for the inner edges (7, 7') of the cranial bone mass and bone flap, **characterized in that** the external support means and internal support means are joined via an intermediate element consisting of a shape-memory material (8), with an initial configuration obtained by subjecting said element to determined requirements, and that once deformed, in order to apply the device between the flap and the cranial bone mass, and upon certain new requirements being applied to it, it adopts the initial configuration.

2. Device for fastening bone fragments (1) according to claim 1, **characterized in that** the external support means (2) and internal support means (6) consist of two platforms (9), which are positioned parallel to one another and joined by a strip of shape-memory material (8) which passes through cuts (10) made in the platforms.

3. Device for fastening bone fragment (1) according to claim 1, **characterized in that** it consists of two bands of shape-memory material (11-11'), joined by a tangential line shared by both bands, outlining two opposite cavities (12-12') for receiving the respective edges of the bone flap (5) and cranial bone mass (4).

4. Device for fastening bone fragments (1) according to claim 1, **characterized in that** the external support means (2) and internal support means (6) consist of two platforms (9), which are positioned parallel to one another and joined by a spring made of a shape-memory material (8), whose ends are adapted to receive respective projections (21) made in the inner surface of the platforms (9).

## Patentansprüche

1. Vorrichtung zum Befestigen von Knochenlappen (1), die verwendet werden, um Knochenfragmente oder -lappen, die während einer chirurgischen Operation von einer Schädelknochenmasse (4) getrennt sind, zu befestigen, wobei wenigstens eine Schädelöffnung (19) definiert ist, wobei die Vorrichtung einige externe Unterstützungsmittel (2) bezüglich der äußeren Kanten (3, 3') der Schädelknochenmasse und des Knochenlappens; und einige innere Unterstützungsmittel (6) bezüglich der inneren Kanten (7, 7') der Schädelknochenmasse und des Knochenlappens umfasst, **dadurch gekennzeichnet, dass** die äußeren Unterstützungsmittel und die inneren Unterstützungsmittel durch ein Zwischenelement miteinander verbunden sind, das aus einem Formerinnerungsmaterial (8) besteht, wobei eine anfängliche Konfiguration durch Beaufschlagen des Elements mit bestimmten Anforderungen erhalten wird, wobei es, sobald es einmal verformt ist, wieder seine anfängliche Konfiguration annimmt, um die Vorrichtung zwischen dem Lappen und der Schädelknochenmasse anzubringen, sobald sie mit bestimmten neuen Anforderungen beaufschlagt wird.

2. Vorrichtung zum Befestigen von Knochenfragmenten (1) nach Anspruch 1, **dadurch gekennzeichnet, dass** die äußeren Unterstützungsmittel (2) und die inneren Unterstützungsmittel (6) aus zwei Plattformen (9) bestehen, die zueinander parallel angeordnet und durch einen Streifen aus einem Formerinnerungsmaterial (8) verbunden sind, der durch Einschnitte (10) verläuft, die in den Plattformen ausgebildet worden sind.

3. Vorrichtung zum Befestigen von Knochenfragmenten (1) nach Anspruch 1, **dadurch gekennzeichnet, dass** es aus zwei Bändern aus einem Formerinnerungsmaterial (11-11') besteht, die durch eine beiden Bändern gemeinsame tangentiale Linie miteinander verbunden sind und die zwei gegenüberliegende Hohlräume (12, 12') auskleiden, um die jeweiligen Kanten des Knochenlappens (5) bzw. der Schädelknochenmasse (4) aufzunehmen.

4. Vorrichtung zum Befestigen von Knochenfragmenten (1) nach Anspruch 1, **dadurch gekennzeichnet, dass** die äußeren Unterstützungsmittel (2) und die inneren Unterstützungsmittel (6) aus zwei Plattformen (9) bestehen, die parallel zueinander angeordnet sind und durch eine Feder verbunden sind, die aus

## Revendications

1. Dispositif pour fixer des volets osseux (1), utilisé pour fixer des fragments ou des volets osseux (5) séparés d'une masse (4) d'os crânien pendant une opération chirurgicale, pendant laquelle au moins un orifice crânien (19) est défini, le dispositif comprenant des moyens de supports externes (2) aux bords externes (3, 3') de la masse d'os crânien et du volet osseux ; et des moyens de support internes (6) pour les bords internes (7,7') de la masse d'os crânien et du volet osseux, **caractérisé en ce que** les moyens de support externes et les moyens de support internes sont reliés *via* un élément intermédiaire consistant en un matériau (8) à mémoire de forme, avec une configuration initiale obtenue en soumettant ledit élément à des exigences déterminées et qui, une fois déformé afin d'appliquer le dispositif entre le volet et la masse d'os crânien, et lorsque certaines nouvelles exigences lui sont appliquées, reprend la configuration initiale.

2. Dispositif pour fixer des fragments osseux (1) selon la revendication 1, **caractérisé en ce que** lesdits moyens de support externes (2) et moyens de support internes (6) consistent en deux plates-formes (9) qui sont positionnées parallèlement l'une à l'autre et reliées par une bande de matériau (8) à mémoire de forme qui passe à travers des découpes (10) réalisées dans les plates-formes.

3. Dispositif pour fixer des fragments osseux (1) selon la revendication 1, **caractérisé en ce qu'**il consiste en deux bandes (11-11') de matériau à mémoire de forme reliées par une ligne tangentielle partagée par les deux bandes, délimitant deux cavités opposées (12-12') pour recevoir les bords respectifs du volet osseux (5) et de la masse (4) d'os crânien.

4. Dispositif pour fixer des fragments osseux (1) selon la revendication 1, **caractérisé en ce que** les moyens de support externes (2) et les moyens de support internes (6) consistent en deux plates-formes (9) qui sont positionnées parallèlement l'une à l'autre et reliées par un ressort fait d'un matériau (8) à mémoire de forme, dont les extrémités sont adaptées pour recevoir des projections respectives (21) faites dans la surface interne des plates-formes (9).
